(19) Europäisches Patentamt
European Patent Office
Office européen des brevets

(11) **EP 4 678 221 A1**

(12) # EUROPEAN PATENT APPLICATION

(43) Date of publication:
14.01.2026   Bulletin 2026/03

(21) Application number: 25183973.4

(22) Date of filing: **19.06.2025**

(51) International Patent Classification (IPC):
**A61N 1/05** (2006.01)        **A61N 1/36** (2006.01)
**A61B 5/00** (2006.01)

(52) Cooperative Patent Classification (CPC):
**A61N 1/0556; A61B 5/4041; A61B 5/6877; A61N 1/36135**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority:  **12.07.2024  US 202418771579**

(71) Applicants:
• **Stichting IMEC Nederland**
  **5656 AE Eindhoven (NL)**

• **The Feinstein Institutes for Medical Research Manhasset, NY 11030 (US)**

(72) Inventors:
• **ROSSETTI, Nicolo**
  **5654 AN Eindhoven (NL)**
• **MIHAJLOVIC, Vojkan**
  **5643 AZ Eindhoven (NL)**
• **ZANOS, Stavros**
  **Manhasset, 11030 (US)**

(74) Representative: **AWA Sweden AB**
**Matrosgatan 1**
**Box 5117**
**200 71 Malmö (SE)**

(54) **SYSTEM AND METHOD FOR NEURAL TISSUE ANATOMY ESTIMATION AND SELECTIVE NEURAL STIMULATION**

(57)    According to an aspect of the present inventive concept there is provided a system for determining an estimate of an anatomy of neural tissue of a subject. The system comprises an electrode arrangement comprising a plurality of electrodes, wherein different electrodes in the plurality of electrodes are configured to be arranged in different electrode locations adjacent the neural tissue, wherein the plurality of electrodes is configured to receive a plurality of stimulation signals, a sensor arrangement configured to generate indications of changes in at least one of a neural activity or a physiological activity in response to the stimulation signals and a processing unit, configured to receive data representing relations between the plurality of stimulation signals and the indications of changes in the neural activity or physiological activity, wherein the processing unit is configured to generate the estimate of the anatomy of the neural tissue of the subject.

Fig. 3

EP 4 678 221 A1

# Description

Technical field

The present description relates to estimating an anatomy of a nerve of a subject and selective stimulation of a nerve of a subject. In particular, the present description relates to a system and method for determining an estimate of an anatomy of neural tissue of a subject, and a method for selective neural stimulation.

Background

Neurostimulation may be used for controlling a function of an organ or part of a body of a living being, such as a human being, e.g., for alleviating or treating a number of conditions of the human being. Neurostimulation may involve a control of electrical signals transported through a nerve, so as to trigger or block signals transported by the nerve.

It is known that peripheral neural fiber properties such as diameter and myelination have an impact on activation thresholds, with larger myelinated fibers being easier to excite as compared to smaller unmyelinated fibers. This poses challenges when trying to recruit smaller fibers before the large ones, due to the different activation thresholds.

However, determining the target fibers for modulating desired organs requires anatomical tracking of fibers innervating that organ. This requires extraction of the complete nerve that innervates target organs, applying immunochemistry imaging to derive anatomical organization of the nerve and tracking the fibers along the nerve. This process is tedious and lengthy and can only be performed postmortem. The information of anatomical neural structure can help in optimizing not only selective activation of fiber populations but also optimizing the dose, minimizing charge delivery, etc., as well as combining several optimization criteria in one.

With anatomical tracking of nerves only being an option postmortem, the current techniques for fitting of a neurostimulation device comprises testing out different stimulating paradigms, until one that provides the least discomfort or provides a desired response is found. This is a lengthy process that may span over weeks or months.

There is a need for a more efficient way of fitting a neurostimulation device and decreasing the discomfort of the subject.

Summary

An objective of the present description is to provide a system and method for estimating an anatomy of neural tissue. Another objective of the description is to provide selective and optimized stimulation of the nerve.

These and other objectives are at least partly met by the invention as defined in the independent claims. Preferred embodiments are set out in the dependent claims.

According to a first aspect, there is provided a system for determining an estimate of an anatomy of neural tissue of a subject, said system comprising an electrode arrangement comprising a plurality of electrodes, wherein different electrodes in the plurality of electrodes are configured to be arranged in different electrode locations adjacent the neural tissue, wherein the plurality of electrodes is configured to receive a plurality of stimulation signals, each stimulation signal being associated with a particular stimulation location adjacent the neural tissue.

The system further comprises a sensor arrangement configured to detect at least one of a neural activity or a physiological activity of the subject, wherein the sensor arrangement is configured to generate indications of changes in at least one of the neural activity or the physiological activity in response to the stimulation signals, and a processing unit, configured to receive data representing relations between the plurality of stimulation signals and the indications of changes in the neural activity or physiological activity, wherein the processing unit is configured to generate the estimate of the anatomy of the neural tissue of the subject using the data received by the processing unit.

The system may be used for determining an estimate of an anatomy of neural tissue of a subject wherein the subject may be a living being. It should be realized that the system may be used for determining an estimate of an anatomy of neural tissue of a human being but that the system may likewise be used for determining an estimate of an anatomy of neural tissue of an animal.

Neural tissue is found in the brain, spinal cord and nerves. As used herein, the term "nerve" should be construed as a bundle of nerve fibers enclosed by a sheath called the epineurium. The nerve may form part of the peripheral nervous system and may therefore also be called a peripheral nerve. The nerve is configured to transmit electrical impulses within the body along axons, which may be bundled in fascicles in the nerve. The electrical impulses may be provided as action potentials transmitted along the axons.

The nerve may comprise a plurality of fascicles. Within the nerve, a large number of fascicles may be provided, and each fascicle may include a large number of axons, such that the nerve may transmit signals to / from various parts of the body. For example, the vagus nerve is a cranial nerve providing signals between the brain and parts of the body. The vagus nerve may be relatively easy to access and a device for stimulating a nerve that is to be implanted in the human being may therefore be advantageously arranged in relation to the vagus nerve for providing stimulation of the vagus nerve. However, since the vagus nerve provides signals to many different parts of the body, it is also important that an estimate of the anatomy is obtained to enable selective stimulation of a desired portion of the nerve for providing a desired effect of the stimulation.

By neural tissue anatomy, or estimate of an anatomy of neural tissue, it is here meant properties of the fascicles within a nerve, or at a larger scale the position/placement of nerves within a bundle of nerves (e.g., in the brain or the spinal cord) and the position of nerve fibers within the nerves of that bundle of nerves. The anatomy of the nerve may in more detail provide the properties of the neurons and/or axons within each of the fascicles. The properties may include the positions of the nerve fibers within the nerve, the spatial trajectories of the nerve fibers, the type of the nerve fibers, or the size of the nerve fibers.

By estimate, it is here meant an approximate calculation of the properties of the nerve fibers or axons within a nerve or neural tissue. The system may represent the estimate of the anatomy in various manners. The estimate of the anatomy may describe spatial arrangement of parts, such as nerve fibers, fascicles, etc., of the nerve or neural tissue. However, the estimate of the anatomy may not necessarily describe an exact spatial arrangement of the parts but may rather describe a map relating functions to spatial positions within the neural tissue. The system may provide a relative map, wherein the relative map may be a representation of the nerve fibers properties relative other fibers and relative the electrodes. The relative map may include relative positions of a fiber in respect to other fibers, relative positions of a fiber in respect to the electrodes and/or the electrode arrangement, and properties, such as type, size, degree of myelination etc., of fibers relative to each other. The estimate may also be referred to as a functional map, wherein the position of the nerve fibers is mapped and categorized based on the physiological response or neural response recorded when stimulated.

The estimate may provide an estimate of absolute spatial arrangement of parts of the neural tissue in the subject. However, the system may not know an exact relation of the electrode arrangement in relation to the neural tissue. For instance, a rotational relation between the electrode arrangement and a nerve may not be known. Thus, the estimate may provide a representation of the anatomy in relation to a coordinate system defined by the electrodes of the electrode arrangement, even though a rotation of such coordinate system within the subject may not be known.

In other words, should the electrode arrangement be rotated or moved, such that the rotational relation between the electrode arrangement and nerve is changed in regard to the estimate of anatomy that has been performed, an additional estimate of anatomy may provide an updated representation of the anatomy in relation to a coordinate system defined by the electrodes of the electrode arrangement.

Stimulation of the nerve may correspond to generation of a sufficiently large action potential so as to trigger a signal to be transported by the nerve or blocking a signal from being transported by the nerve.

The electrode arrangement comprises a plurality of electrodes, and may preferably comprise at minimum two electrodes. With different electrode locations it is here meant that the electrodes are each positioned or placed at a distance from each other having different relations to the neural tissue. A stimulation signal may be provided to a pair of electrodes, such that an electrical field may be generated between the electrodes, wherein the electrical field extends into the neural tissue.

The plurality of electrodes may comprise one or more pre-defined pairs of electrodes to be used for providing the stimulation signal. However, according to an alternative, the electrodes forming a pair of electrodes used for providing a stimulation signal may be dynamically selected among the plurality of electrodes.

In other words, the stimulation may be bipolar, monopolar or tripolar. In one example, using monopolar stimulation, one of the electrodes in a pair of electrodes may be the same electrode for all pairs of electrodes, i.e., all pairs of electrodes share a common electrode. This electrode may be referred to as a reference electrode, and the reference electrode may dynamically form pair with other electrodes of the plurality of electrodes. In another example, using bipolar stimulation, the electrodes are paired up, either as pre-defined pairs of electrodes or dynamically selected pairs of electrodes.

The electrode arrangement is adapted to provide the pairs of electrodes close to or in contact with the neural tissue to be stimulated. This implies that stimulation signals may be output by the electrode arrangement directly into the neural tissue providing an accurate control of the stimulation within the nerve.

It should be appreciated that having a minimum of two electrodes equates to having a minimum of one stimulation location. This provides a simple mapping correlating to that particular stimulation location.

By providing multiple pairs of electrodes, and thus multiple stimulation locations, more advanced and accurate estimates may be generated.

The number of pairs of electrodes and accordingly, stimulation locations, may be decided on a case-to-case basis, and may depend on the neural tissue of interest.

The system or part of the system may be configured to be completely implanted within the body of the subject. This facilitates use of the implanted device for providing stimulation of the nerve to the living being in a convenient way. In particular, when the system or part of the system is implanted within the body of a human being, the stimulation of the nerve may be provided with minimal effect of everyday life of the human being for providing the stimulation.

However, the implanted system or part of the system may still be configured to communicate with an external part of the system through wireless communication.

For example, the electrode arrangement may be implanted, while the other parts of the system (i.e., the sensor arrangement and the processing unit) may be external to the subject's body.

The sensor arrangement may be a portable sensor arrangement, such as a pulse oximeter, heart rate monitor or a smart watch. The sensor arrangement may be hospital equipment for measuring and monitoring different bodily functions of the subject.

The sensor arrangement may be implanted within the body of the subject. Part of the sensor arrangement may be implanted within the body of the subject.

The sensor arrangement may comprise at least one sensor, however it may preferably comprise a plurality of different sensors intended to detect indications of changes of different neural or physiological activity, respectively.

The processing unit may be a wearable processing unit, such as a smart watch, a mobile device, such as a smart phone comprising a processing unit, or a computer.

By physiological response, it is meant a recording or measure of a change in the subject. The physiological response may be a neural response or a response in organ activity, e.g., heart activity, respiratory activity, gastrointestinal activity,

Furthermore, it is possible to derive physiological effects from the elicited neural activity from the same nerve.

By adjacent to it is here meant in close proximity or next to the neural tissue. It is realized that the neural tissue may comprise different geometries depending on the type of neural tissue or location of the neural tissue. For example, when stimulating nerves, such as the vagus nerve, the electrodes may be arranged in different electrode locations adjacent to the outer surface of the nerve, and spaced apart to at least partially surround the circumference of the nerve. In some cases, such as in spinal cord stimulation, the neural structure may more planar and the electrodes may be arranged in different electrode locations adjacent the spinal cord. Hence, the preferred arrangement of electrodes may depend on the neural structure of the neural tissue that is to be stimulated.

The estimate of anatomy may also be referred to or considered a map of the anatomy. The map may be considered a functional map. The estimate of anatomy may include nerve fiber position or location within a nerve or nerve tissue, the thickness, the depth, what type of nerve fibers are present, etc.

By providing an estimate of anatomy of a nerve of a subject selective neural stimulation can be performed based on the estimate of anatomy. By knowing what nerve fibers give what physiological response, the stimulation may be more accurate. Fibers corresponding to physiological responses that may be unwanted or provide negative side effects may be avoided when basing the stimulation on the estimate of nerve anatomy. This is advantageous not only in that the subject will experience less discomfort during stimulation, but also in that selective stimulation demands less stimulation, and is thereby an energy efficient solution.

The present disclosure is advantageous in that it provides a solution to and facilitates targeting of only functionally relevant fibers while minimizing the activation of non-target ones.

Further, system of the present disclosure is advantageous in that the determining of an estimate of anatomy of neural tissue is relatively fast.

The system allows determining an estimate of the anatomy of neural tissue based on a plurality of stimulation signals using a plurality of electrodes. The system thus determines an estimate of the anatomy based on different responses to different stimulation signals. The detected neural activity or physiological activity for the different responses provides input for determining the estimate of the anatomy.

Thanks to the system being configured to determine an estimate of the anatomy of the neural tissue, there is no need to test all possible settings for stimulation signals that may be provided by the electrode arrangement. Rather, a few settings of stimulation signals may be used for estimating the anatomy.

The plurality of stimulation signals may comprise stimulation signals provided using different electrodes in the plurality of electrodes and/or using different amplitudes of the stimulation signals and/or different stimulation pulse shapes. If the electrode arrangement comprises a single pair of electrodes, the plurality of stimulation signals may comprise using a plurality of amplitudes of stimulation signals provided to the pair of electrodes and/or a plurality of stimulation pulse shapes. If the electrode arrangement comprises a plurality of pairs of electrodes, the plurality of stimulation signals may comprise providing a single stimulation signal to each pair of electrodes. Other combinations of stimulation signals may also be used.

The system may be used for determining the estimate of the anatomy of neural tissue when initially arranging the electrode arrangement in relation to the neural tissue. Once the estimate of the anatomy of neural tissue is determined, the estimated anatomy may then be used for calculating or simulating response also to non-tested stimulation signals for determining settings to be used by the system for providing a desired stimulation to the subject using the electrode arrangement.

It should also be realized that the stimulation signals used in determining the estimate of the anatomy of the neural tissue may be the same or different to the stimulation signals that may later be used for providing the desired stimulation to the subject. For instance, the plurality of stimulation signals used for determining the estimate of the anatomy may be based on providing a signal to a single pair of electrodes at a particular point in time. During later stimulation of the subject, stimulation signals may be provided to a plurality of pairs of electrodes for forming interferential stimulation within the neural tissue. However, it should also be realized that the plurality of stimulation signals used for determining the estimate of the anatomy may also include interferential stimulation signals.

According to an embodiment, different electrodes in the plurality of electrodes may be configured to be arranged in different electrode locations around a circumference of a nerve of the neural tissue.

This is advantageous in that having the electrode arrangement, and thereby the stimulation locations, arranged around the circumference of a nerve will provide a more accurate estimate of the anatomy.

The nerve fibers of the nerve may, if only stimulating the nerve from one side, be positioned next to each other, such that an electrical field from the electrodes will affect nerve fibers close to the electrodes in addition to nerve fibers arranged farther away from the electrodes. More than one nerve fiber may be stimulated by a stimulation signal at a particular stimulation location, depending on the properties of the stimulation signal. Due to this, when the electrode arrangement is used for providing desired stimulation of the subject, it may be preferable to stimulate the nerve or nerve tissue from multiple locations around the nerve, to cause interferential stimulation which may provide selective stimulation in a location within the nerve.

According to an embodiment the electrode arrangement may be configured to be implanted in the subject, and wherein the electrode arrangement may be configured to at least partially surround the nerve of the subject.

The electrode arrangement may for example be ring shaped or shaped as a cylinder having a through hole and an opening intended to be threaded around a nerve.

In another example the electrode arrangement is wrapped around the nerve.

By partially surround the nerve of the subject it is here meant that the electrode arrangement may at least partly enclose the nerve, in other words, the electrode arrangement may, in some embodiments, not extend around the full circumference of the nerve. The electrode arrangement may comprise an opening or the like, and thereby partly surround the nerve.

The electrode arrangement may in one example be in the form of a cuff configured to be arranged surrounding the circumference of the nerve, wherein the plurality of electrodes is arranged on said cuff. The cuff may fully surround the nerve or have an opening such that the nerve is only partially surrounded by the cuff.

The cuff provides a convenient manner of arranging the electrodes in relation to a cross-section of the nerve. The cuff may be easily mounted around the nerve, whereby the cuff will position the electrodes of the first pair of electrodes and the second pair of electrodes in appropriate positions in relation to the nerve. This facilitates implantation of the device.

According to an embodiment, the different electrodes in the plurality of electrodes are configured to be arranged in different electrode locations adjacent to the spinal cord or adjacent to brain tissue.

Thus, the system may be used for determining an estimate of anatomy of neural tissue of the spinal cord or brain.

According to an embodiment, the estimate of the anatomy of the nerve represents properties of nerve fibers within the nerve, and wherein the properties of the nerve fibers within the nerve comprises at least one of the positions of the nerve fibers within the nerve, the spatial trajectories of the nerve fibers, the type of the nerve fibers, or the size of the nerve fibers.

The properties of the nerve fibers of the nerve may be estimated based on the indication of change in neural activity or physiological activity detected by the sensor arrangement, in relation to the stimulation parameters of the stimulation signal. The spatial arrangement of the nerve fibers within the nerve may be estimated based on relating a stimulation signal to a particular pair of electrodes in a particular location in relation to the neural tissue to a particular change in neural activity or physiological activity.

In addition, a higher current amplitude may give rise to a different change in physiological or neural activity, than a lower current amplitude. This may, at least in part, be due to the properties of the nerve fibers, and thus the relation can be established.

According to an embodiment, the physiological activity of the subject may be at least one of a physiological activity indicative of heart activity, a physiological activity indicative of lung activity, a physiological activity indicative of muscle activity, a physiological activity indicative of gastrointestinal activity, a physiological activity indicative of urinary activity, and a physiological activity indicative of genital activity.

Which physiological activity that is of interest may depend on what neural tissue is observed. For example, the vagus nerve controls sensory functions to the outer ear, throat, heart, and abdominal organs, motor functions in the throat and parasympathetic functions such as heart rhythm, gastric movement, and respiratory functions. Other nerves may control other functions. Similarly, different parts of the spine controls different functions and different parts of brain control different functions.

Hence, by knowing which nerve is being stimulated, and thus for which nerve the anatomy is intended to be estimated, it can be determined which physiological activities is needed to be detected, i.e., which sensors needs to be comprised in the sensor arrangement.

It should be appreciated that the physiological activity may be determined using the same sensor arrangement or different sensor arrangements. A physiological activity indicative of heart activity may for example be blood pressure, heart rate or electrocardiography. A physiological activity indicative of lung activity may be the respiration rate.

It should be appreciated that the aforementioned physiological responses are purely examples given to aid in the understanding of the present disclosure. The physiological responses may be anything related to the body and bodily functions of the subject.

Furthermore, the neural activity may be a neural response. The neural responses may be measured by sensors placed in the vicinity of nerves being stimulated or in the vicinity of organs.

By measuring at least one physiological activity it is possible to determine locations of nerve fibers in the nerve, providing a particular physical response.

According to an embodiment, the system further comprises a data storage, wherein the data storage is configured to store nerve anatomy data sets and wherein the processing unit is further configured to generate the estimate of the anatomy of the neural tissue of the subject by comparing the received data with stored nerve anatomy data sets.

The data storage may be a part of the processing unit, or the data storage may be a separate unit. The data storage may be a wearable data storage, such as a smart watch, a mobile device, such as a smart phone comprising a data storage, or a computer.

For example, in the case of a wearable, such as a smart watch, both the processing unit and the data storage may form part of the smart watch.

The processing unit may be adapted to retrieve information from the storage unit. The processing unit may be adapted to store information in the data storage.

The data storage may comprise previously determined estimates of anatomy of neural tissue.

The data storage may further comprise nerve anatomy data sets retrieved postmortem.

The processing unit may, after receiving data representing relations between the plurality of stimulation signal and the indications of changes in the neural activity or physiological activity, generate an estimate of anatomy of neural tissue, by comparing the received data with the data stored in the data storage.

The nerve anatomy data sets may include a representation of a spatial arrangement of nerve fibers in the neural tissue, spatial trajectories of the nerve fibers, and/or size of the nerve fibers. The nerve anatomy data sets may also link the representation of the spatial arrangement of nerve fibers in the neural tissue to responses to stimulation signals. The nerve anatomy data sets may be based on previous tests performed for other subjects, wherein stimulation signals are provided using electrodes arranged in relation to neural tissue. In addition, the nerve anatomy data sets may also include histological images, which may be obtained postmortem, such as to provide a detailed representation of the spatial arrangement of the nerve fibers.

The nerve anatomy data sets may also or alternatively comprise synthetically determined responses to stimulation signals for a known anatomy of neural tissue. Thus, using a model for propagation of electrical signals in the neural tissue, an effect of stimulation signals for a known anatomy may be computed or simulated. Hence, even if no actual tests of responses to stimulation signals have been performed for a particular anatomy, such responses may be synthetically determined and stored in the nerve anatomy data sets.

The nerve anatomy data sets may thus comprise information of expected responses to stimulation signals based on an anatomy of the neural tissue. By determining responses to stimulation signals for a subject for which the estimate of the anatomy of neural tissue is to be performed, recorded responses may be compared to responses for the nerve anatomy data sets in order to find a nerve anatomy data set within the data storage providing a close match to the recorded responses. This may be used for accurately determining the representation of the nerve fibers in the neural tissue of the subject.

The system may thus be configured to initially determine a functional map relating functional responses to stimulation signals provided by different electrodes in the electrode arrangement. The nerve anatomy data sets may also comprise functional maps. The comparing of the received data with stored nerve anatomy data sets may comprise comparing functional maps to each other in order to find a functional map in the stored nerve anatomy data sets corresponding to the recorded functional map for the subject.

Since the nerve anatomy data sets may comprise an accurate representation of anatomy of neural tissue, e.g., based on histological images, it is thus possible to determine similar estimations for the subject based on a selected nerve anatomy data set, and in that way determine an estimation of the anatomy of the neural tissue of the subject.

According to an example the data storage may be configured to store functional mapping data sets. Nerve anatomy data sets may include functional mapping data sets. The processing unit may be configured to generate the estimate of the anatomy of the neural tissue of the subject by comparing the received data with stored nerve anatomy data sets and/or the functional mapping data sets.

According to an embodiment, the processing unit may be configured to compute a functional mapping using a relation between indications of changes in the neural activity or the physiological activity and the stimulation locations, wherein the processing unit is configured to generate the estimate of the anatomy of the neural tissue based on the functional mapping.

The functional map may in itself be considered an estimate of anatomy of neural tissue, as the functional map illustrates the stimulation locations and the physiological activity arisen from stimulation at a certain stimulation location.

Further, the functional map may be used for comparison to the anatomy data sets stored in the data storage, and thereby aid in finding an accurate match in a data storage providing relations between functional maps and anatomies.

In other words, there is provided multiple options on determining an estimate of an anatomy of neural tissue.

For example, the estimate may be determined solely based on the indications of changes in neural of physiological activity in relation to the properties of the stimulation signal and the stimulation locations.

As a further example, the estimate may be determined based on the indications of changes in neural of physiological activity in relation to the properties of the stimulation signal and the stimulation locations, and by comparing this with existing anatomy information retrieved from the data storage.

In a further example, a functional map is generated, and estimate of anatomy is either derived directly from the functional map or derived by comparing the functional map to functional maps and their corresponding estimates of anatomy or anatomy data sets of the data storage.

According to an embodiment, the plurality of electrodes of the electrode assembly may comprise pairs of electrodes, and wherein each pair of electrodes may comprise a first electrode and a second electrode configured to be displaced along a longitudinal axis of the nerve of the subject.

Thus, a stimulation signal may be provided using electrodes displaced along a longitudinal direction of the nerve. This may imply that detected responses may be related to a particular circumferential position of the electrodes. Hence, a cross-section of the anatomy of the nerve may be determined, wherein the position of the longitudinally displaced electrodes may be represented by a single point at a circumference of the cross-section.

According to an embodiment, the first electrode and the second electrode are configured to generate an electrical field therebetween upon receiving the stimulation signals.

The electric field may cause a stimulation of a nerve fiber in the neural tissue triggering a signal to be transported by the nerve fiber or blocking a signal from being transported by the nerve fiber.

According to an embodiment, the processing unit may be further configured to receive updated data representing relations between the plurality of stimulation signals and the indications of changes in the neural activity or the physiological activity, wherein the processing unit may be configured to determine an updated relation between the electrode arrangement and the neural tissue using the estimate of the anatomy of the neural tissue of the subject.

By providing an adaptive system, having the possibility to update the data representing relations between the plurality of stimulation signals and the indications of changes in the neural activity and/or the physiological activity as the relations change, the system may allow updating a relation between the electrode arrangement and the neural tissue.

Thus, if the electrode arrangement is displaced, e.g., rotated in relation to the nerve, the system may determine an updated relation between the electrode arrangement and the neural tissue, to allow stimulations provided by the electrode arrangement to be continued. Hence, if the electrode arrangement is displaced, there may not be a need for replacing or adjusting the position of the electrode arrangement, and surgery may be avoided.

According to a second aspect, there is provided a method for determining an estimate of an anatomy of neural tissue of a subject, said method comprising the steps of stimulating, using an electrode arrangement, a plurality of stimulation locations of the neural tissue of the subject, detecting, using a sensor arrangement, indications of changes in at least one of a neural activity or a physiological activity, receiving, at a processing unit, data representing relations between the stimulation signals, the stimulation locations and the indications of changes in the neural activity or the physiological activity, and generating an estimate of the anatomy of the neural tissue of the subject.

This aspect may generally present the same or corresponding advantages as the first aspect.

Effects and features of this second aspect are largely analogous to those described above in connection with the first aspect.

Embodiments mentioned in relation to the second aspect are largely compatible with the first aspect.

The aforementioned method may be implemented by a system comprising an electrode arrangement, a sensor arrangement and a processing unit. For example, the method may be implemented using a system such as the system according to a first aspect of this disclosure.

The method relates to determining an estimate of an anatomy of neural tissue. The method stimulates a plurality of stimulation locations of the neural tissue of the subject and detects indications of change in neural activity or physiological activity as a result of said stimulation. The method further receives data related to the stimulation and detection and determines an estimated of an anatomy of the neural tissue of the subject.

It should be appreciated that as neural tissue includes the brain, the spinal cord and nerves, the above includes these options. For example, the method may be understood as a method for determining an estimate of an anatomy of a nerve of a subject.

By stimulating one particular stimulation location at the time, the indication of change in neural activity or physiological activity may be directly correlated to that stimulation location. Further to this, properties related to the nerve fiber at the stimulation location may be determined based on the properties of the stimulation signal and the detected indications of change. Thereby, an estimate of an anatomy may be generated.

According to another aspect, a computer-implemented method for determining an estimate of an anatomy of neural tissue of a subject comprises: receiving, at a processing unit, data representing relations between stimulation signals, stimulation locations and indications of changes in the neural activity or the physiological activity; and generating an estimate of the anatomy of neural tissue of the subject, based on the received data.

Thus, the method may relate to generating an estimate of the anatomy based on received data. The method may include any of the features described above in relation to the first aspect for generating the estimate based on the received data.

According to an embodiment, the estimate of the anatomy of the neural tissue of the subject represents properties of nerve fibers within the neural tissue in relation to the electrode arrangement and corresponding to the change in the neural activity or the physiological activity for each nerve fiber.

According to another aspect, there is provided a computer program product comprising computer-readable instructions such that when executed on a processing unit the computer program product will cause the processing unit to perform the aforementioned computer-implemented method for determining an estimate of an anatomy of neural tissue of a subject.

Effects and features of this aspect are largely analogous to those described above in connection with the first and second aspects. Embodiments mentioned in relation to this aspect are largely compatible with the first and second aspects.

The computer program product may implement the method in a processing unit, which may be a dedicated processing unit for performing the method or may be a general-purpose processing unit which may be able to perform the method based on the computer program product. For instance, the processing unit may be embedded in a small, implantable apparatus.

The computer program product may comprise a computer-readable medium on which the computer-readable instructions are stored. The computer program product may thus be provided as a non-transient computer program product stored on any tangible medium. Alternatively, the computer program product may be provided as a signal carrying the computer-readable instructions for allowing the computer program product to be loaded into a memory accessible to the processing unit.

According to a third aspect, there is provided a computer-implemented method for controlling selective neural stimulation, comprising the steps of receiving a computational anatomical model, wherein the computational anatomical model comprises an estimate of the anatomy of neural tissue of a subject, wherein the estimate is determined according to second aspect, determining, based on the computational anatomical model and on information of electrode locations of a plurality of electrodes arranged in different electrode locations adjacent the neural tissue, a stimulation parameter set for the plurality of electrodes for stimulation of at least one target fiber, wherein the target fiber is a fiber of the neural tissue of the subject corresponding to a desired change in a neural activity or a physiological activity.

This aspect may generally present the same or corresponding advantages as the first and the second aspect.

Effects and features of this third aspect are largely analogous to those described above in connection with the first and second aspects.

Embodiments mentioned in relation to the third aspect are largely compatible with the first and second aspects.

The computer-implemented method relates to controlling selective neural stimulation. The computer-implemented method determines a stimulation parameter set, based on computational anatomical model that is, at least in part, comprised of an estimate of the anatomy of a nerve of a subject. The stimulation parameter set may be used when stimulating at least one target fiber of the neural tissue.

The method does not relate to actual stimulation, i.e., actual output of pulses, and is therefore not related to a method of treatment of a living being.

The at least one target fiber corresponds to a desired change in a neural activity or a physiological activity. A desired change may for instance be a specific motor function, sensory function or parasympathetic response. A desired change may also be the absence or removal of neural or physiological activity. This could for example be the removal of muscle tremor.

This is made possible by the stimulation parameter set, and the stimulation location, which is chosen in regard to the computational anatomical model comprising an estimate of an anatomy of the neural tissue.

Advantageously, the method may with high accuracy provide a determination of a stimulation parameter set, which may then be used for stimulating the chosen target fiber(s), while decreasing and/or minimizing unwanted neural and physiological responses, as the selection is based on a subject specific neural anatomy mapping.

Furthermore, the method may advantageously decrease the amount of stimulation needed. In other words, by providing a method for controlling selective neural stimulation, with high accuracy in determining stimulation parameter sets that may be used for causing stimulation of target nerve fibers, the amount of stimulation using the stimulation parameter sets may be minimized. That is, the number of electrodes activated, as well as the energy needed to reach the desired effect may be individually and precisely determined and applied.

According to an embodiment, the computer-implemented method may further comprise outputting the stimulation parameter set to a control unit for triggering output of a stimulation signal using the stimulation parameter set to selected electrodes in the plurality of electrodes for causing stimulation of the at least one target fiber.

The computer-implemented method outputs the determined stimulation parameter set. A control unit receives the outputted stimulation parameter set.

In one example, the processing unit may comprise the control unit. In other words, the processing unit and the control unit may be the same. In a further example, the processing unit and the control unit are separate units.

According to an embodiment, determining the stimulation parameter set may comprise calculating the number of off-target fibers and target fibers activated by a candidate stimulation parameter set, and determining the stimulation parameter set for avoiding stimulation of off-target fibers, when stimulating the at least one target fiber.

This may be a suitable manner of determining the stimulation parameter set to provide a control of stimulation that may cause stimulation of a target fiber or a set of target fibers while not stimulating undesired, off-target fibers.

According to an embodiment, the stimulation parameter set comprises at least one of a pair of selected electrodes, a current amplitude, a pulse shape, a frequency, a duration time of the stimulation, a pulse repetition frequency, or a pulse width.

According to another aspect, there is provided a computer program product comprising computer-readable instructions such that when executed on a processing unit the computer program product will cause the processing unit to perform the aforementioned computer-implemented method for controlling selective neural stimulation.

Effects and features of this aspect are largely analogous to those described above in connection with the previously described aspects. Embodiments mentioned in relation to this aspect are largely compatible with the first and second aspects.

The computer program product may implement the method in a processing unit, which may be a dedicated processing unit for performing the method or may be a general-purpose processing unit which may be able to perform the method based on the computer program product. For instance, the processing unit may be embedded in a small, implantable apparatus.

The computer program product may comprise a computer-readable medium on which the computer-readable instructions are stored. The computer program product may thus be provided as a non-transient computer program product stored on any tangible medium. Alternatively, the computer program product may be provided as a signal carrying the computer-readable instructions for allowing the computer program product to be loaded into a memory accessible to the processing unit.

According to a fourth aspect of the present disclosure, there is provided a system according to the first aspect of the present disclosure, wherein the processing unit is configured to perform the computer-implemented method according to the third aspect of the present disclosure.

This aspect may generally present the same or corresponding advantages as the first, the second and the third aspects.

Effects and features of this third aspect are largely analogous to those described above in connection with the first, second and third aspects.

Embodiments mentioned in relation to the third aspect are largely compatible with the first, second and third aspects.

The processing unit may have separate functions for generating the estimate of the anatomy of the neural tissue and for determining the stimulation parameter set. These functions may be implemented in a single processor or may be implemented in a plurality of processors forming a distributed processing unit.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a schematic view illustrating a system for determining an estimate of an anatomy of neural tissue of a subject.
Fig. 2 is a schematic view of generation of the estimate of the anatomy.
Fig. 3 is a schematic view of a system for determining the estimate of the anatomy and for determining a stimulation parameter set for providing stimulation of the neural tissue.
Fig. 4 schematically illustrates a method for determining an estimate of the anatomy of neural tissue of a subject.
Fig. 5 schematically illustrates a computer-implemented method for selective neural stimulation

Detailed description

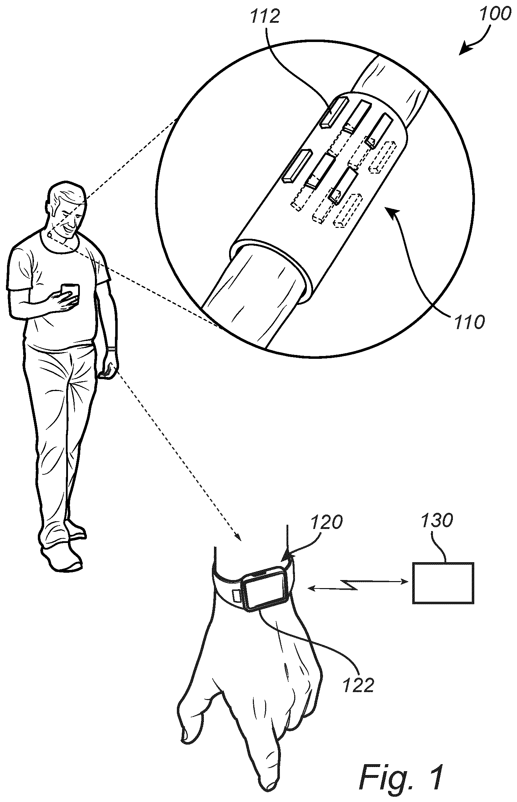

Referring now to Fig. 1, a system 100 for determining an estimate of an anatomy of neural tissue of a subject.

As illustrated in Fig. 1, the system 100 comprises an electrode arrangement 110, a sensor arrangement 120 and a processing unit 130.

The electrode arrangement 110 may comprise a plurality of electrodes. The electrodes are preferably arranged in pairs.

The processing unit 130 may be configured to communicate with the electrode arrangement 110. The processing unit 130 may for instance be configured to provide control signals for controlling a stimulation signal to be output by the electrode arrangement 110.

The processing unit 130 may be configured to communicate with the sensor arrangement 120. For example, the processing unit 130 may be configured receive the detected changes in physiological activity and/or neural activity from the sensor arrangement 120. The processing unit 130 may further be configured to send instructions to the sensor arrangement 120 regarding what physiological activity should be detected.

The processing unit 130 may communicate with the sensor arrangement 120 wirelessly or by wired connection.

In a preferred embodiment, the sensor arrangement 120 and the processing unit 130 communicated wirelessly, by e.g., wi-fi, Bluetooth, or similar.

The processing unit 130 may be implemented as a processing unit that executes a software program for performing the method. According to an alternative, the processing unit may be implemented as a dedicated hardware circuitry, such as an Application-Specific Integrated Circuit (ASIC).

The electrode arrangement 110 is configured to be implanted in a subject. The subject may be a human or animal. The electrode arrangement 110 may be implanted at the brain, spine or around any nerve in the body of the subject. The electrodes 112 of the electrode arrangement 110 are arranged in different electrode locations adjacent the nerve or neural tissue of interest.

As illustrated in Fig. 1, the electrodes 112 of the electrode arrangement 110 may comprise a plurality of pairs of electrodes 112, wherein each pair comprises two electrodes displaced longitudinally in relation to each other along a nerve at which the electrode arrangement 110 is arranged. The electrode arrangement 110 may thus be arranged in a cuff, which is arranged around the nerve for placing the electrodes 112 in relation to the nerve.

The electrode arrangement 110 may comprise a plurality of pairs of electrodes 112 arranged in different circumferential positions around the nerve. In Fig. 1, six pairs of electrodes 112 are illustrated arranged in six different locations around the nerve.

It should be realized that in other embodiments, other numbers of electrodes may be used and that a larger or a smaller number of electrodes may be used. In a simple embodiment, the electrode arrangement comprises a single pair of electrodes.

Each of the pairs of electrodes form a stimulation location, wherein a stimulation location is established between the first and the second electrode of the pair of electrodes.

The sensor arrangement 120 may comprise a single sensor 122. This sensor 122 is configured to detect a change in a physiological activity or a neural activity in response to the stimulation of the electrodes. The sensor 122 may be configured to detect change in a single physiological activity or a single neural activity or may be configured to detect changes in more than one physiological activity and/or neural activity.

The processing unit 130 is configured to receive, from the sensor 122 of the sensor arrangement 120, data representing relations between stimulation signals and indications of changes in the physiological activity or neural activity.

The stimulation signal comprises properties, for example, a pair of selected electrodes, a current amplitude, a frequency, a duration time of the stimulation, a pulse repetition frequency, and/or a pulse width. These properties of the stimulation signal are related to the detected change in physiological activity and/or neural activity.

In a very specific example, the sensor 122 may be a heart rate monitor. The first pair of electrodes are activated, and thereby stimulating a specific stimulation location with a stimulation signal having specific properties, listed above.

If the sensor 122, i.e., pulse monitor, detects a change in the pulse of the subject, this will be received by the processing unit 130 and thus a change in physiological activity is received by the processing unit 130 in relation to the stimulation location and stimulation signal.

The data received by the processing unit 130 can thus represent the change in physiological activity and/or neural activity in relation to the stimulation location and properties of the stimulation signal that caused the change in physiological activity and/or neural activity.

In other words, stimulation by the same pairs of electrodes, at the same stimulation location with different stimulation properties may gave rise to different changes in physiological and/or neural activity.

The system 100 may be used for providing a plurality of stimulation signals and detecting neural activity and/or physiological activity of the subject in response to the stimulation signals. By detecting the response from different stimulation signals, a relation between stimulation signals and changes in physiological activity and/or neural activity is determined which may be used for generating an estimate of the anatomy of the neural tissue.

If a single pair of electrodes is used, different stimulation signals may be provided to the pair of electrodes for determining the relation between changes in physiological activity and/or neural activity. However, the processing unit 130 may only be able to generate a rough estimate based on such stimulation.

In the set-up of Fig. 1, stimulation signals may be provided to the six different pairs of electrodes 112. While providing stimulation signals, different physiological responses may be determined using one or more sensors 122 of the sensor arrangement 120.

The responses detected may depend on the neural tissue being stimulated. In an example, the electrode arrangement 110 is arranged in relation to a vagus nerve, which comprises nerve fibers related with various different physiological functions. The sensors 122 may be configured to measure how strong effect is provided on different physiological functions based on stimulation signals being provided by different electrodes 112 in the electrode arrangement 110. Based on such measurements, a radar chart may be determined representing strength of response in physiological function in relation to stimulation by the electrodes 112 in different circumferential positions around the nerve. Such radar chart may thus provide a functional map of the anatomy of the nerve. However, it should be realized that the relation between the stimulation signals and responses in physiological activity may be represented in different manners. In addition, a neural activity, such as a signal in a nerve distal to a position at which stimulation is provided, may be detected in addition to, or instead of, physiological activity.

In the example of stimulation of the vagus nerve, the sensors 122 may be configured to detect changes in breathing rate, laryngeal EMG response, and heart rate. Thus, the stimulation of the vagus nerve may be related to effects to lungs, laryngeal muscle, and heart. It should be realized that other responses may also or alternatively be sensed, such as a physiological activity indicative of gastrointestinal activity, a physiological activity indicative of urinary activity, or a physiological activity indicative of genital activity.

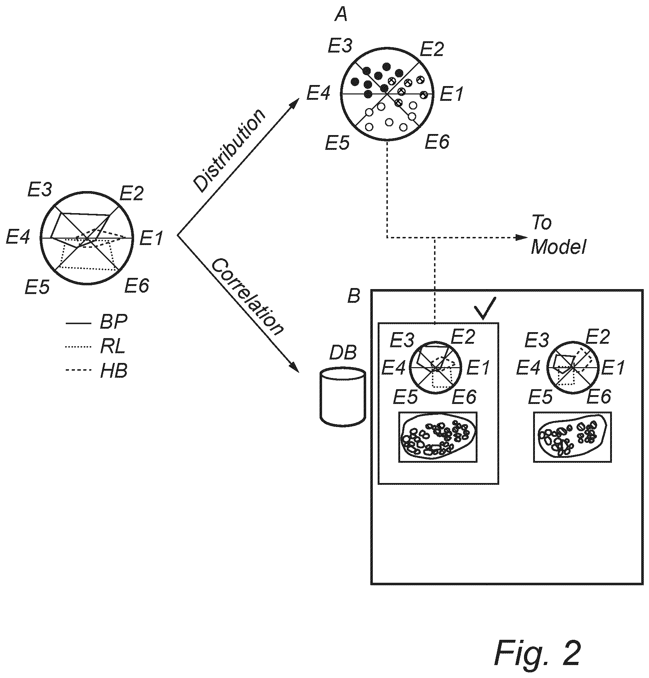

Referring now to Fig. 2, the processing unit 130 may be configured to determine a functional map or functional relation between stimulation signals to specific stimulation locations and responses in physiological or neural activity, as represented by the radar chart illustrated in Fig. 2, wherein symbols E1-E6 represent locations around the circumference of the nerve of six electrode pairs and the radar chart indicates effects to lungs (BP), laryngeal muscle (RL), and heart (HB), respectively.

The processing unit 130 may further be configured to generate the estimate of the anatomy of the neural tissue using the functional map. The processing unit 130 may be configured to generate the estimate of the anatomy by directly determining an estimated map of position of different functional fiber groups within the nerve. This may be determined based on information of which stimulation locations provide which functional effects, as illustrated in part A of Fig. 2.

However, according to an alternative, the processing unit 130 has access to nerve anatomy data sets. The nerve anatomy data sets may be stored in a data storage 140 which is accessible to the processing unit 130.

The nerve anatomy data sets may comprise functional maps corresponding to the functional map determined for the subject. The nerve anatomy data sets may further comprise detailed information of the anatomy of nerves.

The processing unit 130 may be configured to compare recorded data from the sensor arrangement 120, as for example represented by the radar chart, to corresponding data within the nerve anatomy data sets in order to find a nerve anatomy data set that corresponds to the recorded data, such as by finding a nerve anatomy data set with a highest correlation to the recorded data.

The nerve anatomy data set may further comprise detailed information of the anatomy, which may thus be used by the processing unit 130 for generating the estimate of the anatomy of the nerve. The processing unit 130 may be configured to use the anatomy of the nerve anatomy data set as a representation of the anatomy of the nerve of the subject or may be configured to further process the anatomy of the nerve anatomy data set to fit the estimate of the anatomy to differences between the recorded data and the corresponding nerve anatomy data set.

A database of nerve anatomy data sets may be determined based on existing implants of electrodes for stimulation of nerves and physiological responses determined based on stimulation signals, in addition to information of nerve anatomy, which may for instance be acquired by histological images postmortem. It should also be realized that the responses may be determined by simulation of stimulation signals to a known anatomy, if recording of responses are not available.

The generated estimate of the anatomy of the nerve may represent a relation between the electrode arrangement 110 and the nerve. The system may further be configured to monitor changes in the relation between the electrode arrangement 110 and the nerve. Thus, the system may be configured to regularly determine updated data representing relations between the plurality of stimulation signals and indications in changes in physiological activity and/or neural activity. The updated data may be used for determining whether there is a need to determine an updated relation and, if so, determine such updated relation by updating the estimate of the nerve anatomy in relation to the position of the electrode arrangement 110. This may be used for enabling the system to handle changes in the arrangement of the electrode arrangement 110 in the subject, such as rotation of a cuff of the electrode arrangement 110 in relation to the nerve.

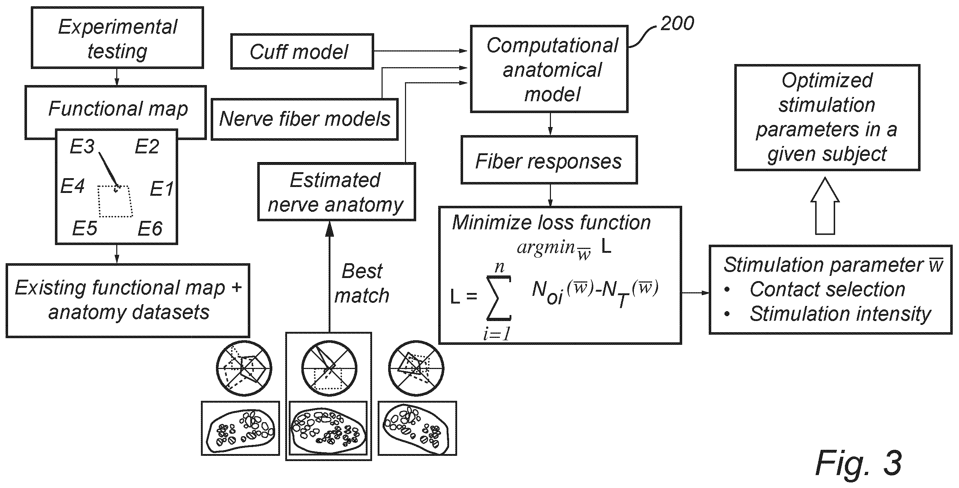

Referring now to Fig. 3, the generated estimate of the anatomy of the nerve may be used for controlling neural stimulation using the electrode arrangement 110. In Fig. 3, the generation of the estimate is illustrated in blocks according to an embodiment.

As shown in Fig. 3, experimental testing may be performed for determining responses to stimulation signals. Based on the testing, a functional map may be determined, which may be further compared to existing functional maps and anatomy data sets for determining a best match. This may be used for estimating the anatomy of the nerve.

The estimate of the anatomy of the nerve may be provided to a computational anatomical model, which is configured to compute an electrical field in the nerve as result of stimulation signals. In addition to the estimate of the anatomy, the computational anatomical model receives a model of the electrode arrangement 110 providing a relation of the electrodes 112 to the anatomy of the nerve. Further, the computational anatomical model receives as input nerve fiber models providing electrical properties of nerve fibers.

The computational anatomical model 200 may be used for determining a response to stimulation signals being provided to the electrodes 112 in the electrode arrangement 110. Thus, by determining the estimate of the anatomy of the nerve in relation to which the electrode arrangement 110 is arranged, the computational anatomical model 200 may be used for determining the stimulation signals to be used for obtaining desired responses from the stimulation of the nerve.

The computational anatomical model 200 may be used for automatically identifying a best set of electrodes 112 and stimulation parameters, such as current amplitude, frequency, duration time of stimulation, pulse repetition frequency, and pulse width, for achieving the desired response.

Optimization criteria may be used based on minimizing a loss function, such that, e.g., a least number of non-target fibers are activated while most of target fibers are activated. The loss function may be defined as:

$$L = \sum_{i=1}^{n} N_{O_i}(\overline{w}) - N_T(\overline{w}),$$

where i represents a function and there are *n* different functions, $N_{O_i}(\overline{w})$ represents a percentage of off-target fibers for function i that are activated during stimulation using a set of parameters $\overline{w}$, while $N_T(\overline{w})$ is a percentage of target fibers that are activated using the same set of parameters $\overline{w}$, and $\overline{w}$ is a vector of size M where M is a total number of current sources (electrode pairs), containing weights of each source and having values bound between 0 (inactive source) and maximum current amplitude.

The optimization needs only to compute a static electric field for each of the electrodes 112 once after construction of the computational anatomical model. Thereafter, the weight vector may be used for generating the final electrical field using superposition of effects from different electrodes 112. Similarly, temporal profiles of the electric field at each fiber location can be obtained based on type of stimulation used. The resulting electrical field is then used for obtaining the fiber responses to stimulation, allowing values of $N_{O_i}(\overline{w})$ and $N_T(\overline{w})$ to be determined as well as allowing the loss function to be determined.

The parameter space of available parameters for the stimulation by the electrodes may then be searched for finding the stimulation parameter set that minimizes the loss function. Then, stimulation of the nerve may be provided through the electrodes using the determined stimulation parameter set.

It should be appreciated that the above is provided as an example and that other optimization criteria may be considered and/or combined with the above loss function. For example, other cost functions may be defined that minimize the total stimulation energy, number of contacts used, etc.

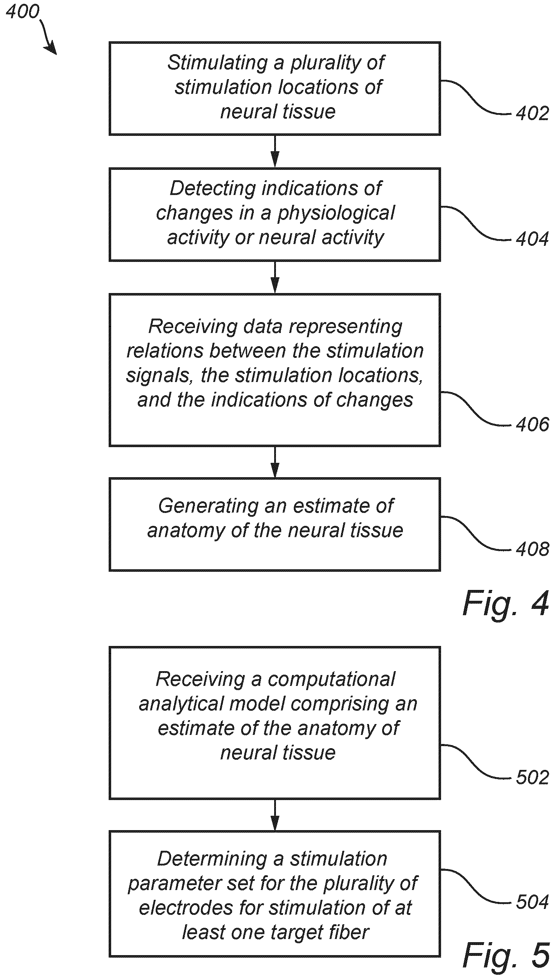

Referring now to Fig. 4, a schematical illustration of a method 400 for determining an estimate of the anatomy of neural tissue of a subject is shown.

As indicated by block 402, the method 400 comprises stimulating, using an electrode arrangement, a plurality of stimulation locations of neural tissue of a subject.

Thereafter, as indicated by block 404, the method 400 further comprises, detecting using a sensor arrangement, indications of changes in a physiological activity or a neural activity.

Once indications of changes in physiological or neural activity have been detected, the method 400 further comprises receiving at a processing unit, data representing relations between the stimulation signals, the stimulation locations and the indications of changes in the physiological activity or the neural activity, as shown by block 406.

Lastly, the method 400 comprises the step of generating an estimate of the anatomy of neural tissue of the subject, based on the received data, as indicated by block 408.

Fig. 5 schematically illustrates a computer-implemented method 300 for controlling selective neural stimulation.

As indicated by block 502, the method 500 comprises receiving a computational anatomical model, wherein the computational anatomical model comprises an estimate of the anatomy of neural tissue of a subject. The estimate of the anatomy is determined according to the aforementioned method 400.

Thereafter, as indicated by block 504, the method 500 further comprises, determining, based on the computational anatomical model and on information of electrode locations of a plurality of electrodes adjacent the neural tissue, a stimulation parameter set for the plurality of electrodes for stimulation of at least one target fiber, wherein the target fiber is a fiber of the nerve of the subject corresponding to a desired change in a physiological characteristic.

The stimulation parameter set may include, for example, a current amplitude, a frequency, a duration time of the stimulation, a pulse repetition frequency, a pulse shape and/or a pulse width.

Itemized list of embodiments

1. A system for determining an estimate of an anatomy of neural tissue of a subject, said system comprising:

an electrode arrangement comprising a plurality of electrodes, wherein different electrodes in the plurality of electrodes are configured to be arranged in different electrode locations adjacent the neural tissue, wherein the plurality of electrodes is configured to receive a plurality of stimulation signals, each stimulation signal being associated with a particular stimulation location adjacent the neural tissue;
a sensor arrangement configured to detect at least one of a neural activity or a physiological activity of the subject, wherein the sensor arrangement is configured to generate indications of changes in at least one of the neural activity or the physiological activity in response to the stimulation signals; and
a processing unit, configured to receive data representing relations between the plurality of stimulation signals and the indications of changes in the neural activity or physiological activity, wherein the processing unit is configured to generate the estimate of the anatomy of the neural tissue of the subject using the data received by the processing unit.

2. The system according to embodiment 1, wherein different electrodes in the plurality of electrodes are configured to be arranged in different electrode locations around a circumference of a nerve of neural tissue.

3. The system according to embodiment 2, wherein the electrode arrangement is configured to be implanted in the subject, and wherein the electrode arrangement is configured to at least partially surround the nerve of the subject.

4. The system according to embodiment 1, wherein the different electrodes in the plurality of electrodes are configured to be arranged in different electrode locations adjacent to the spinal cord or adjacent to brain tissue.

5. The system according to any one of the preceding embodiments, wherein the estimate of the anatomy of the nerve represents properties of nerve fibers within the nerve, and wherein the properties of the nerve fibers within the nerve comprises at least one of

the positions of the nerve fibers within the nerve,
the spatial trajectories of the nerve fibers,
the type of the nerve fibers, or
the size of the nerve fibers.

6. The system according to any one of the preceding embodiments, wherein the physiological activity of the subject is at least one of:

a physiological activity indicative of heart activity,

a physiological activity indicative of lung activity,
a physiological activity indicative of muscle activity,
a physiological activity indicative of gastrointestinal activity,
a physiological activity indicative of urinary activity, or
a physiological activity indicative of genital activity.

7. The system according to any one of the preceding embodiments, further comprising a data storage, wherein the data storage is configured to store nerve anatomy data sets and wherein the processing unit is further configured to generate the estimate of the anatomy of the neural tissue of the subject by comparing the received data with stored nerve anatomy data sets.

8. The system according to any one of the preceding embodiments, wherein the processing unit is configured to compute a functional mapping using a relation between indications of changes in the neural activity or the physiological activity and the stimulation locations, wherein the processing unit is configured to generate the estimate of the anatomy of the neural tissue based on the functional mapping.

9. The system according to any one of the preceding embodiments, wherein the plurality of electrodes of the electrode assembly comprises pairs of electrodes, and wherein each pair of electrodes comprises a first electrode and a second electrode configured to be displaced along a longitudinal axis of the nerve of the subject.

10. The system according to embodiment 9, wherein the first electrode and the second electrode are configured to generate an electrical field therebetween upon receiving the stimulation signals.

11. The system according to any one of the preceding embodiments, wherein the processing unit is further configured to receive updated data representing relations between the plurality of stimulation signals and the indications of changes in the neural activity or the physiological activity, wherein the processing unit is configured to determine an updated relation between the electrode arrangement and the neural tissue using the estimate of the anatomy of the neural tissue of the subject.

12. A method for determining an estimate of an anatomy of neural tissue of a subject, said method comprising the steps of

stimulating, using an electrode arrangement, a plurality of stimulation locations of the neural tissue of the subject;

detecting, using a sensor arrangement, indications of changes in at least one of a neural activity or a physiological activity;

receiving, at a processing unit, data representing relations between the stimulation signals, the stimulation locations and the indications of changes in the neural activity or the physiological activity; and

generating an estimate of the anatomy of the neural tissue of the subject, based on the received data.

13. The method according to embodiment 12, wherein the estimate of the anatomy of the neural tissue of the subject represents properties of nerve fibers within the neural tissue in relation to the electrode arrangement and corresponding to the change in the neural activity or physiological activity for each nerve fiber.

14. A computer-implemented method for determining an estimate of an anatomy of neural tissue of a subject, comprising the steps of

receiving, at a processing unit, data representing relations between stimulation signals, stimulation locations and indications of changes in neural activity or physiological activity, and

generating an estimate of the anatomy of the neural tissue of the subject, based on the received data.

15. A computer program product comprising computer-readable instructions such that when executed on a processing unit the computer program product will cause the processing unit to perform the computer-implemented method of embodiment 14.

16. A computer-implemented method for controlling selective neural stimulation, comprising the steps of

receiving a computational anatomical model, wherein the computational anatomical model comprises an estimate of the anatomy of neural tissue of a subject, wherein the estimate is determined according to the method of embodiment 12-15,

determining, based on the computational anatomical model and on information of electrode locations of a plurality of electrodes arranged in different electrode locations adjacent the neural tissue, a stimulation parameter set for the plurality of electrodes for stimulation of at least one target fiber, wherein the target fiber is a fiber of the neural tissue of the subject corresponding to a desired change in a physiological activity or neural activity.

17. The computer-implemented method according to embodiment 16, further comprising outputting the stimulation parameter set to a control unit for triggering output of a stimulation signal using the stimulation parameter set to selected electrodes in the plurality of electrodes for causing stimulation of the at least one target fiber.

18. The computer-implemented method according to any one of embodiments 16-17, wherein determining the stimulation parameter set comprises calculating the number of off-target fibers and target fibers activated by a candidate stimulation parameter set, and determining the stimulation parameter set for avoiding stimulation of off-target fibers, when stimulating the at least one target fiber.

19. The computer-implemented method according to any one of embodiments 16-18, wherein the stimulation parameter set comprises at least one of

a pair of selected electrodes,
a current amplitude,
a pulse shape,
a frequency,
a duration time of the stimulation
a pulse repetition frequency, or
a pulse width.

20. A computer program product comprising computer-readable instructions such that when executed on a processing unit the computer program product will cause the processing unit to perform the computer-implemented method of any one of embodiments 16-19.

21. A system according to any one of embodiments 1-11, wherein the processing unit is configured to perform the computer-implemented method according to any one of embodiments 16-19.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A system for determining an estimate of an anatomy of neural tissue of a subject, said system comprising:

an electrode arrangement comprising a plurality of electrodes, wherein different electrodes in the plurality of electrodes are configured to be arranged in different electrode locations adjacent the neural tissue, wherein the plurality of electrodes is configured to receive a plurality of stimulation signals, each stimulation signal being associated with a particular stimulation location adjacent the neural tissue; a sensor arrangement configured to detect at least one of a neural activity or a physiological activity of the subject, wherein the sensor arrangement is configured to generate indications of changes in at least one of the neural activity or the physiological activity in response to the stimulation signals; and a processing unit, configured to receive data representing relations between the plurality of stimulation signals and the indications of changes in the neural activity or physiological activity, wherein the processing unit is configured to generate the estimate of the anatomy of the neural tissue of the subject using the data received by the processing unit.

2. The system according to claim 1, wherein different electrodes in the plurality of electrodes are configured to be arranged in different electrode locations around a circumference of a nerve of neural tissue.

3. The system according to claim 2, wherein the electrode arrangement is configured to be implanted in the subject, and wherein the electrode arrangement is configured to at least partially surround the nerve of the subject.

4. The system according to any one of the preceding claims, wherein the estimate of the anatomy of the nerve represents properties of nerve fibers within the nerve, and wherein the properties of the nerve fibers within the nerve comprises at least one of

   the positions of the nerve fibers within the nerve, the spatial trajectories of the nerve fibers, the type of the nerve fibers, or the size of the nerve fibers.

5. The system according to any one of the preceding claims, wherein the physiological activity of the subject is at least one of:

   a physiological activity indicative of heart activity, a physiological activity indicative of lung activity, a physiological activity indicative of muscle activity, a physiological activity indicative of gastrointestinal activity, a physiological activity indicative of urinary activity, or a physiological activity indicative of genital activity.

6. The system according to any one of the preceding claims, further comprising a data storage, wherein the data storage is configured to store nerve anatomy data sets and wherein the processing unit is further configured to generate the estimate of the anatomy of the neural tissue of the subject by comparing the received data with stored nerve anatomy data sets.

7. The system according to any one of the preceding claims, wherein the processing unit is configured to compute a functional mapping using a relation between indications of changes in the neural activity or the physiological activity and the stimulation locations, wherein the processing unit is configured to generate the estimate of the anatomy of the neural tissue based on the functional mapping.

8. The system according to any one of the preceding claims, wherein the plurality of electrodes of the electrode assembly comprises pairs of electrodes, and wherein each pair of electrodes comprises a first electrode and a second electrode configured to be displaced along a longitudinal axis of the nerve of the subject.

9. The system according to claim 8, wherein the first electrode and the second electrode are configured to generate an electrical field therebetween upon receiving the stimulation signals.

10. The system according to any one of the preceding claims, wherein the processing unit is further configured to receive updated data representing relations between the plurality of stimulation signals and the indications of changes in the neural activity or the physiological activity, wherein the processing unit is configured to determine an updated relation between the electrode arrangement and the neural tissue using the estimate of the anatomy of the neural tissue of the subject.

11. A computer-implemented method for controlling selective neural stimulation, comprising the steps of

    receiving a computational anatomical model, wherein the computational anatomical model comprises an estimate of the anatomy of neural tissue of a subject, determining, based on the computational anatomical model and on information of electrode locations of a plurality of electrodes arranged in different electrode locations adjacent the neural tissue, a stimulation parameter set for the plurality of electrodes for stimulation of at least one target fiber, wherein the target fiber is a fiber of the neural tissue of the subject corresponding to a desired change in a physiological activity or neural activity.

12. The computer-implemented method according to claim 11, further comprising outputting the stimulation parameter set to a control unit for triggering output of a stimulation signal using the stimulation parameter set to selected electrodes in the plurality of electrodes for causing stimulation of the at least one target fiber.

13. The computer-implemented method according to any one of claims 11-12, wherein determining the stimulation parameter set comprises calculating the number of off-target fibers and target fibers activated by a candidate stimulation parameter set, and determining the stimulation parameter set for avoiding stimulation of off-target fibers, when stimulating the at least one target fiber.

14. The computer-implemented method according to any one of claims 11-13, wherein the stimulation parameter set comprises at least one of a pair of selected electrodes,

a current amplitude,
a pulse shape
a frequency,
a duration time of the stimulation
a pulse repetition frequency, or
a pulse width.

15. A computer program product comprising computer-readable instructions such that when executed on a processing unit the computer program product will cause the processing unit to perform the method according to any one of claims 11-14.

Fig. 1

*Fig. 2*

Fig. 3

400

```
┌─────────────────────────────┐
│   Stimulating a plurality of │
│   stimulation locations of   │──── 402
│        neural tissue         │
└─────────────────────────────┘
              │
              ▼
┌─────────────────────────────┐
│   Detecting indications of   │
│  changes in a physiological  │──── 404
│  activity or neural activity │
└─────────────────────────────┘
              │
              ▼
┌─────────────────────────────┐
│   Receiving data representing│
│ relations between the stimulation│
│  signals, the stimulation locations,│──── 406
│  and the indications of changes │
└─────────────────────────────┘
              │
              ▼
┌─────────────────────────────┐
│     Generating an estimate of │
│    anatomy of the neural tissue │──── 408
└─────────────────────────────┘
```

*Fig. 4*

```
┌─────────────────────────────┐
│   Receiving a computational  │
│  analytical model comprising an│
│   estimate of the anatomy of │──── 502
│        neural tissue         │
└─────────────────────────────┘
              │
              ▼
┌─────────────────────────────┐
│   Determining a stimulation  │
│ parameter set for the plurality of│
│ electrodes for stimulation of at│──── 504
│     least one target fiber   │
└─────────────────────────────┘
```

*Fig. 5*

Europäisches
Patentamt

European
Patent Office

Office européen
des brevets

## EUROPEAN SEARCH REPORT

**Application Number**

EP 25 18 3973

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/198111 A1 (BEUTEL FABIAN [DE]) 20 June 2024 (2024-06-20) * paragraphs [0010], [0018], [0095], [0098] - [0105], [0110], [0124], [0146] - [0148], [0163], [0164]; figures 1-3 * | 1-15 | INV. A61N1/05 A61N1/36 A61B5/00 |
| A | US 2004/006281 A1 (MATSUKAWA KANJI [JP] ET AL) 8 January 2004 (2004-01-08) * the whole document * | 1-15 | |
| A | US 9 055 875 B2 (TRIANTIS IASONAS [GB]; TOUMAZOU CHRISTOFER [GB]; VAGONYX LTD [VG]) 16 June 2015 (2015-06-16) * the whole document * | 1-15 | |

**TECHNICAL FIELDS SEARCHED (IPC)**

A61N
A61B

The present search report has been drawn up for all claims

| Place of search | Date of completion of the search | Examiner |
|---|---|---|
| Munich | 8 December 2025 | Pfeiffer, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 3973

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2025

| Patent document cited in search report | | Publication date | Patent family member(s) | | Publication date |
|---|---|---|---|---|---|
| US 2024198111 | A1 | 20-06-2024 | EP | 4389193 A1 | 26-06-2024 |
| | | | US | 2024198111 A1 | 20-06-2024 |
| US 2004006281 | A1 | 08-01-2004 | NONE | | |
| US 9055875 | B2 | 16-06-2015 | CN | 101489473 A | 22-07-2009 |
| | | | CN | 103211589 A | 24-07-2013 |
| | | | DK | 2037802 T3 | 24-09-2012 |
| | | | EP | 2037802 A2 | 25-03-2009 |
| | | | EP | 2374408 A1 | 12-10-2011 |
| | | | US | 2009292345 A1 | 26-11-2009 |
| | | | WO | 2008007065 A2 | 17-01-2008 |

EPO FORM P0459

For more details about this annex : see Official Journal of the European Patent Office, No. 12/82